# EUROPEAN PATENT APPLICATION

(11) **EP 3 552 605 A1**
(43) Date of publication of application: **16.10.2019**
(21) Application number: 18166780.9
(22) Date of filing: 11.04.2018
(51) Int. Cl.: A61K 31/4545, A61P 17/00, A61P 43/00, A61P 25/00, A61P 35/00, A61P 9/00

(54) **MCOPPB FOR USE AS MEDICAMENT**

(71) Applicant: Univerzita Palackého V Olomouchi, 77147 Olomouc (CZ); Smart Brain S.r.o., 14220 Praha 4 (CZ)
(72) Inventor: Mistrik, Martin, 78301 Olomouc (CZ); Hajduch, Marian, 79305 Moravsky Beroun (CZ); Taborska, Natalie, 77900 Olomouc (CZ); Bartek, Jiri, 2670 Greve (DK)
(74) Representative: Hartvichova, Katerina

(57) **Abstract**

The present invention provides 1-[1-(1-methylcyclooctyl)-4-piperidinyl]-2-[(3R)-3-piperidinyl]-1H-benzimidazole of formula 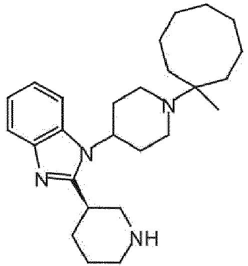 for use in a method of treatment of a condition involving accumulation of senescent cells. The conditions involving accumulation of senescent cells are in particular cancer, cardiovascular, neurology and immune related diseases.

## Description

### Field of Art

The invention relates to a novel use of 1-[1-(1-methylcyclooctyl)-4-piperidinyl]-2-[(3R)-3-piperidinyl]-1H-benzimidazole (MCOPPB), for the elimination of senescent cells from living animal organisms, in particular mammals including humans.

### Background Art

MCOPPB (1-[1-(1-methylcyclooctyl)-4-piperidinyl]-2-[(3R)-3-piperidinyl]-1H-benzimidazole) is a drug which acts as a potent and selective non-peptide agonist for the nociceptin receptor (pKi = 10.07 ± 0.01) and has a much weaker activity on other opioid receptors. It has only moderate affinity for the mu opioid receptor, weak affinity for the kappa opioid receptor and negligible binding at the delta opioid receptor. In animal studies, MCOPPB produces potent anxiolytic effects, with no inhibition of memory or motor function, and only slight sedative side effects which do not appear until much higher doses than the effective anxiolytic dose range.

In an ex vivo binding study, MCOPPB (10 mg/kg, p.o.) inhibited signaling through the NOP receptor in the mouse brain, suggesting that it penetrated into the brain after it was orally administered. In the mouse Vogel conflict test, MCOPPB (10 mg/kg, p.o.) and diazepam (3 mg/kg, p.o.) elicited anxiolytic-like effects, although MCOPPB produced a bell-shaped response curve. In addition, MCOPPB (10 mg/kg, p.o.) was still effective as an anxiolytic agent even after repeated administration for 5 days. MCOPPB at an oral dose of 10 mg/kg did not affect locomotor activity or memory, nor did it contribute to ethanol-induced hypnosis. On the other hand, the benzodiazepine-type anxiolytic agent diazepam caused memory deficits and enhanced ethanol-induced hypnosis. These findings suggest that MCOPPB - a compound with few adverse effects on the central nervous system - is a potential therapeutic agent for the treatment of anxiety.

Cellular senescence is defined as permanent growth arrest promoted by permanent DNA damage signaling and is generally percept as defense mechanism of the body to eliminate genomically unstable cells (cells with damaged DNA) from cell cycle and thus prevent the onset of diseases such as cancer. On the other hand, accumulation of senescent cells has also negative impact on the organism including age-related pathologies such as atherosclerosis or arthritis and may promote tumorigenesis, by creating a tumor-promoting microenvironment fueled by the secreted cytokines. Indeed, there are multiple studies including mice-based GMO systems allowing selective killing of the senescent cells *in-vivo* proving that elimination of senescent cells might be beneficial for the organism in multiple aspects and generally improves the fitness of animal models (Nat Med. 2016 Jan;22(1):78-83, Clin Pharmacol Ther. 2013 Jan;93(1):105-16., Nature. 2011 Nov 2;479(7372):232-6). Elimination of senescent cells seems to be particularly beneficial in later stages of ontogenesis (advanced age) as this life period is typically guided by accumulation of large amounts of senescent cells.

WO2015157738 discloses the use of opioid analgesics for relieving pain and other undesired side effects in cancer treatments. MCOPPB is mentioned as an example of a synthetic opioid. However, this document is not concerned with the treatment of the cancer as such or of aging in general, it only discloses relieving side effects connected with cancer treatment by exploiting the analgesic effects of the opioids.

### Disclosure of the Invention

The present invention is based on the unexpected finding that the MCOPPB is capable of selectively eliminating senescent cells.

In a first aspect, the present invention thus provides 1-[1-(1-methylcyclooctyl)-4-piperidinyl]-2-[(3R)-3-piperidinyl]-1H-benzimidazole (abbreviated MCOPPB) for use in the treatment of conditions involving accumulation of senescent cells.

The MCOPPB has the structural formula

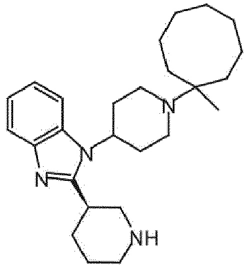

The term "MCOPPB" is to be interpreted as including the compound in the form of a free base or in the form of salts and/or solvates. Preferred salts are pharmaceutically acceptable acid addition salts, such as hydrochlorides. Most preferred is the trihydrochloride salt.

The conditions involving accumulation of senescent cells preferably include advanced age-related disorders caused by natural accumulation of senescent cells, inherited premature aging syndromes, disorders caused by aberrant accumulation of senescent cells during ontogenesis of the organism, accumulation of senescent cells induced by environmental poisoning, accumulation of senescent cells induced by chemotherapy, local accumulation of senescent cells in tissues and organs induced by therapeutic or accidental irradiation by ionizing radiation, accumulation of senescent cells in the skin induced by UV light, accumulation of senescent cells induced by oncogenic transformation of cells or other environmental factors.

The conditions involving accumulation of senescent cells are preferably selected from cancer, cardiovascular, neurologic and immune disorders, such as atherosclerosis or arthritis.

The senescent cells may be of different tissue origins including muscle cells, skin cells, haematopoietic cells, neural cells, stem cells, cancer cells. The senescence in the cells may be induced by various factors including natural aging-related factors, heritage syndromes, toxic elements in the environment, chemotherapy induced, ionizing radiation induced, UV light induced, and induced by oncogenic transformation of the cell.

The compound of the invention may be used to eliminate or kill senescent cells within the organism with minimum adverse effects as manifested by good health of tested animals including positive weight score and normal behavior.

In a second aspect of the present invention, a method for treatment of conditions involving accumulation of senescent cells is provided, comprising the step of administering a therapeutically effective amount of MCOPPB to a patient suffering from a condition involving accumulation of senescent cells. The patient is preferably a mammal, more preferably a human.

MCOPPB may be administered, for example, at a dose of about 1-100 mg/kg/day, preferably 5-50 mg/kg/day, more preferably 10-20 mg/kg/day. Preferably, the MCOPPB is administered perorally, but other modes of administration may be suitable as well.

### Definitions and Pharmaceutical Formulations:

The invention provides a method for treating conditions caused by accumulation of senescent cells including cancer prevention, the method comprising administering to a patient in need thereof a therapeutically effective amount of a formulation of the invention. By analogy, it is also within the scope of the invention to use the formulation of the invention in the manufacture of a medicament for the treatment of disorders caused by accumulation of senescent cells.
As used herein, the term "condition" means a condition of deteriorated health of an organism, or an abnormal condition of a part, organ or organism. The term "condition" encompasses abnormal health states, illnesses, disorders and diseases.
It will be appreciated that the term "treatment" and "treating" as used herein means the management and care of a patient for the purpose of combating or alleviating a condition, such as a disease or a disorder. The term is intended to include the full spectrum of treatments for a given condition from which the patient is suffering, such as administration of the active compound to slow down the progress of the condition such as a disorder or a disease, to cure or eliminate the disorder or condition as well as to prevent the condition, wherein prevention is to be understood as the management and care of a patient for the purpose of combating the senescent cells induced disorder and includes the administration of the active compounds to prevent the onset of the symptoms or complications. The patient to be treated is preferably a mammal, in particular a human being, but it may also include animals, such as dogs, cats, horses, cows, goats, sheep and pigs.

The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995.
Suitable pharmaceutical carriers include inert solid diluents or fillers, sterile aqueous solutions and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospholipids, fatty acids, fatty acid amines, polyoxyethylene and water. In addition, the compound of the invention may form solvates with water or common organic solvents. Such solvates are also encompassed within the scope of the present invention.
The composition may further comprise a buffer system, preservative(s), tonicity agent(s), chelating agent(s), stabilizers and surfactants, which is well known to the skilled person. For convenience reference is made to Remington: The Science and Practice of Pharmacy, 20th edition, 2000. The composition may also further comprise one or more therapeutic agents active against the same disease state. Methods to produce controlled release systems useful for compositions of the current invention include, but are not limited to, crystallization, condensation, co-crystallization, precipitation, co-precipitation, emulsification, dispersion, high pressure homogenisation, en-capsulation, spray drying, microencapsulating, coacervation, phase separation, solvent evaporation to produce microspheres, extrusion and supercritical fluid processes. General reference is made to Handbook of Pharmaceutical Controlled Release (Wise, D. L. , ed. Marcel Dekker, New York, 2000) and Drug and the Pharmaceutical Sciences vol. 99: Protein Composition and Delivery (MacNally, E.J. , ed. Marcel Dekker, New York, 2000) .
Administration of pharmaceutical compositions according to the invention may be through several routes of administration, for example, oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route. It will be appreciated that the preferred route will depend on the general condition and age of the subject to be treated, the nature of the condition to be treated and the active ingredient chosen. For topical use, sprays, creams, ointments, jellies, gels, inhalants, dermal patches, implants, solutions of suspensions, etc., containing the compound of the present invention may be contemplated. For the purpose of this application, topical applications shall include mouth washes and gargles. Compound of the invention may be used in wafer technology, such as the biodegradable Gliadel polymer wafer, which is very useful for brain cancer chemotherapy.
Pharmaceutical compositions for oral administration include solid dosage forms such as hard or soft capsules, tablets, troches, draggers, pills, lozenges, powders and granules; and liquid dosage forms for oral administration which include solutions, emulsions, aqueous or oily suspensions, syrups and elixirs, each containing a predetermined amount of the active ingredient, and which may include a suitable excipient. Compositions intended for oral use may be prepared according to any known method, and such compositions may contain one or more agents selected from the group consisting of sweetening agents, flavoring agents, coloring agents, and preserving agents in order to provide pharmaceutically elegant and palatable preparations.
Tablets may contain the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patents 4,356,108; 4,166,452; and 4,265,874 to form osmotic therapeutic tablets for controlled release.
Formulations for oral use may also be presented as hard gelatine capsules where the active ingredient is mixed with an inert solid diluent, for example, calcium carbonate, calcium phosphate or kaolin, or as soft gelatine capsules wherein the active ingredient is mixed with water or an oil medium, for example peanut oil, liquid paraffin, or olive oil.
Aqueous suspensions may contain the active compound in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.
Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.
Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present. The pharmaceutical compositions of the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.
Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be in the form of a sterile injectable aqueous or oleaginous suspension. This suspension may be formulated according to known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as a solvent or suspending medium. For this purpose, any bland fixed oil may be employed using synthetic mono- or diglycerides. In addition, fatty acids such as oleic acid find use in the preparation of injectables.
Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a solution or suspension for the administration of the active compound in the form of a nasal or pulmonal spray. As a still further option, the pharmaceutical compositions containing the compound of the invention can also be adapted to transdermal administration, e.g. by needle-free injection or from a patch, optionally an iontophoretic patch, or transmucosal, e.g. buccal, administration. Pharmaceutical compositions for parenteral administration include sterile aqueous and non-aqueous injectable solutions, dispersions, suspensions or emulsions as well as sterile powders to be reconstituted in sterile injectable solutions or dispersions prior to use. The term "aqueous composition" is defined as a composition comprising at least 50% w/w water. Likewise, the term "aqueous solution" is defined as a solution comprising at least 50% w/w water, and the term "aqueous suspension" is defined as a suspension comprising at least 50% w/w water. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art. Depot injectable formulations are also contemplated as being within the scope of the present invention.
Generally, depending on the intended mode of administration, the formulation will contain about 0.005% to 95%, preferably about 0.5% to 50%, by weight of the active ingredient. The percentage of active compound contained in the formulation is dependent on the specific nature of the formulation, as well as the needs of the subject. Percentages of active ingredient of 0.01% to 90% in solution are generally employable, whilst the amounts can be higher if the formulation is a solid which will be subsequently diluted. In some embodiments, the formulation will comprise from 1 % to 50% of the active compound in solution.
The use of a formulation according to the invention will generally be in an amount effective to achieve the intended result, for example in an amount effective for selective removal of senescent cells. The formulation may be administered therapeutically to achieve therapeutic benefit. By therapeutic benefit is meant eradication or amelioration of the underlying disorder being treated and/or eradication and/or amelioration of one or more of the systems associated with the underlying disorder.
The exact dosage will depend upon the frequency and mode of administration, the sex, age, weight and general condition of the subject treated, the nature and severity of the condition treated and any concomitant diseases to be treated and other factors evident to those skilled in the art.
In one preferred embodiment, the formulation will take the form of a unit dosage form. For example, the formulation may be provided in a vial or other container. The vial or other container may contain the formulation in the form of a liquid, a solid to be suspended, a dry powder, a lyophilisate, or any other suitable form.

The invention will be further illustrated by means of non-limiting examples, with references to the following figures.

### Brief Description of Figures

Figure 1 is a micrograph of GIEMSA stained ARPE-1 cells: parental cells (normal healthy ARPE-1 cell population) and senescent cells, each untreated and treated by MCOPPB. Senescent and normal ARPE-1 cells were either mock treated (left column) or exposed to 10µM MCOPPB for 48 hours (right column). Differential toxicity is manifested by cell deterioration and cell loss in the MCCOPB treated senescent cells (lower right corner). (Example 1)
Figure 2 represents a chart summarizing differential toxicity of various concentrations of MCOPPB towards the normal (parental) and senescent ARPE-1 cells. (Example 1)
Figure 3 is a micrograph of GIEMSA stained MRC5 cells. Senescent and normal (parental) MRC5 cells were either mock treated or exposed to 10µM MCOPPB for 48 hours. Differential toxicity is manifested by cell deterioration and cell loss in the MCCOPB treated senescent cells. (Example 2)
Figure 4 represents a chart summarizing the mean weight and standard deviation of animals treated by MCOPPB at indicated concentration. Stars mark the application days. (Example 3)
Figure 5 shows the whole body luminescent signal of a 12 month old p16 reporter mouse before (left) and the same mouse after three weeks of MCOPPB treatment (right). Treated mouse shows a rapid decrease of the signal (darker color) evidencing the disappearance of the senescent cells.
Figure 6 represents charts of whole body luminescent signals of 12-16 months old p16 reporter mice treated either by vehicle (vehiculum) or by MCOPPB for two weeks. MCOPPB treated mice show a decrease of the luminescence signal.

### Examples of Carrying Out the Invention

### Materials used:

### Chemicals:

MCOPPB (Sigma Aldrich)
Aphidicolin (Sigma Aldrich)
GIEMSA stain (Abcam)
MTT Cell Proliferation Assay kit (ATCC)
SA-Beta-Gal Senescence Detection Kit (Abcam, ab65351)

### Cell lines:

hTERT RPE-1 human retinal epithelial cell line was obtained from ATTC. Cells were grown in DMEM medium (Sigma Aldrich) supplemented with 10% fetal bovine serum (FBS) and antibiotics (penicillin-streptomycin).
MRC-5 human lung fibroblast cell line was obtained from ATTC. Cells were grown in DMEM (Sigma Aldrich) supplemented with 10% FBS and antibiotics (penicillin-streptomycin).

### Example 1: Preferential toxicity of MCOPPB towards human ARPE-1 senescent cells

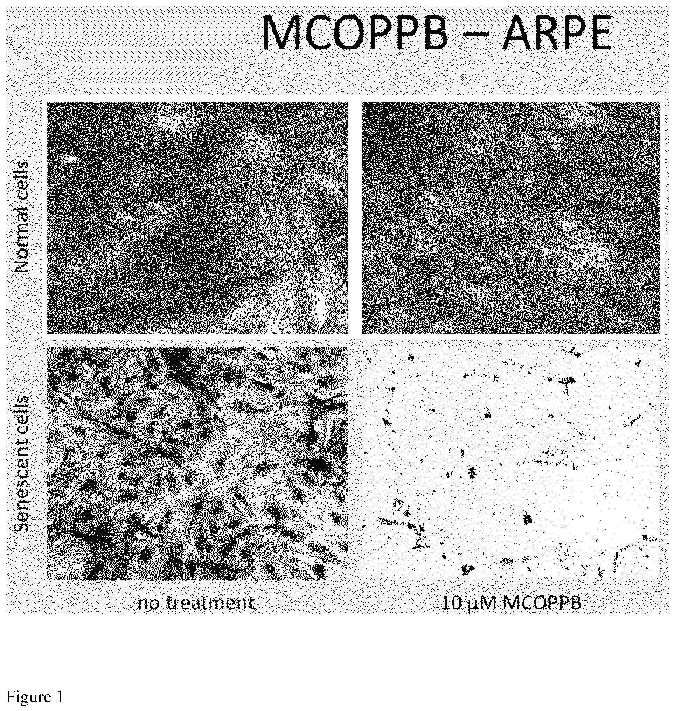

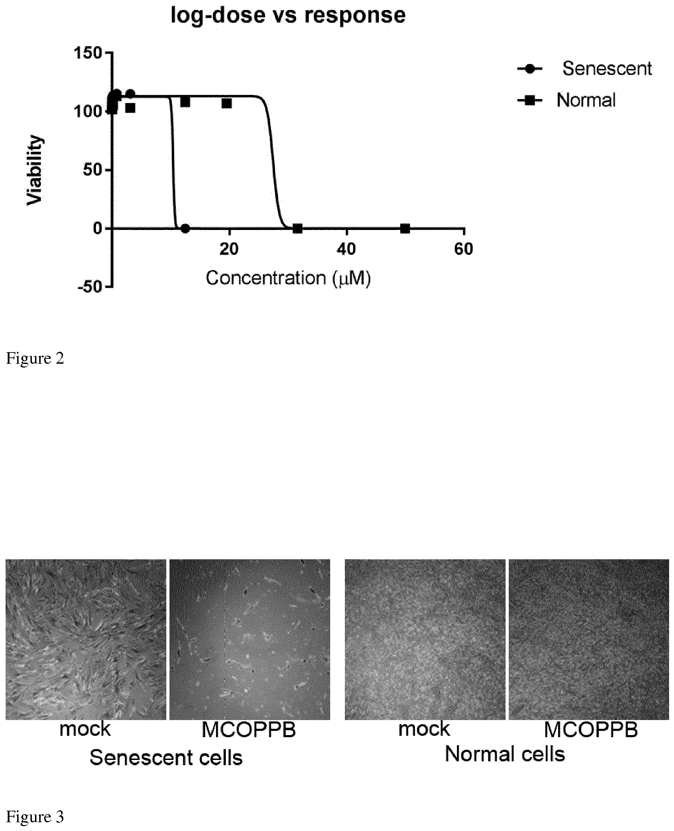

In human ARPE-1 human epithelial cells, senescence response - manifested by loss of proliferation, typical morphological changes and Senescence Associated Beta-galactosidase positivity - was induced by exposition to replication stress for prolonged time period. The induction involved 200nM aphidicolin continual treatment for three weeks. The senescent cells have been exposed to various doses of MCOPPB dissolved in water for 48 hours, the dose range spanned 100 nM - 50 µM. The number of surviving cells was evaluated immediately after the treatment either using GIMSA staining and microscopy-based evaluation of surviving cells and compared to parental exponentially growing ARPE-1 cells, which were exposed to MCOPPB as well. Control samples of senescent and parental cells, without MCOPPB treatment, were assessed, too. In Figure 1, the effect of selective toxicity of 10 µM MCOPPB compound towards the senescent cells is visually demonstrated (micrograph of GIEMSA stained cells). It should be noted that no adverse effects on the parental (normal healthy) ARPE-1 cells were observed at this concentration. Figure 2 depicts the cytotoxic effect of various MCOPPB concentrations on the senescent and the parental (normal, exponentially growing) ARPE-1 cells using 48h exposition followed by immediate MTT cytotoxic test performed according manufacturer's instructions.

### Example 2: Preferential toxicity of MCOPPB towards human MRC5 senescent cells

In human MRC-5 fibroblasts cells, senescence response - manifested by loss of proliferation, typical morphological changes and Senescence Associated Beta-galactosidase positivity - was induced by exposition to gamma radiation. The induction involved a single irradiation, total dose 15 Gy using Xstrahl RS 225 M RTG device set to 200KV 15mA (dose 3.8Gy/min). The senescent cells have been exposed to various doses of MCOPPB dissolved in water for 48 hours, the dose range spanned 100-10µM. The number of surviving cells was evaluated immediately after the treatment either using GIMSA staining and microscopy-based evaluation of surviving cells and compared to parental exponentially growing MRC-5 cells. In Figure 3, is visually demonstrated (micrograph of GIEMSA stained cells) the effect of selective toxicity of 5 µM MCOPPB compound towards the senescent cells is visually demonstrated (micrograph of GIEMSA stained cells).

### Example 3: Tolerance of long term application of MCOPPB in-vivo

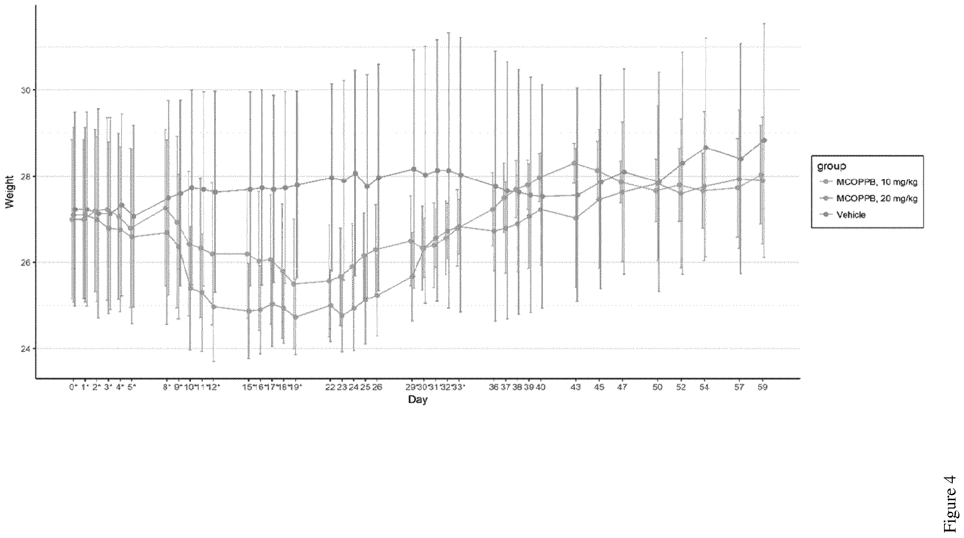

Female mice (C57B1/6 strain) at the age of 42 weeks were treated by intraperitoneal application of MCOPPB (10 mg/kg and 20 mg/kg) dissolved in phosphate buffer or by the buffer alone (vehicle) for five weeks in the regime 5 + 2 (5 days with the treatment, two days without) and followed for another two weeks. The weight and another physiological factors reflecting overall fitness were monitored. Both concentrations were well tolerated by the animals (see figure 4 for the mice weight evolution during the experiment)

### Example 4: Elimination of senescent cells by MCOPPB in-vivo

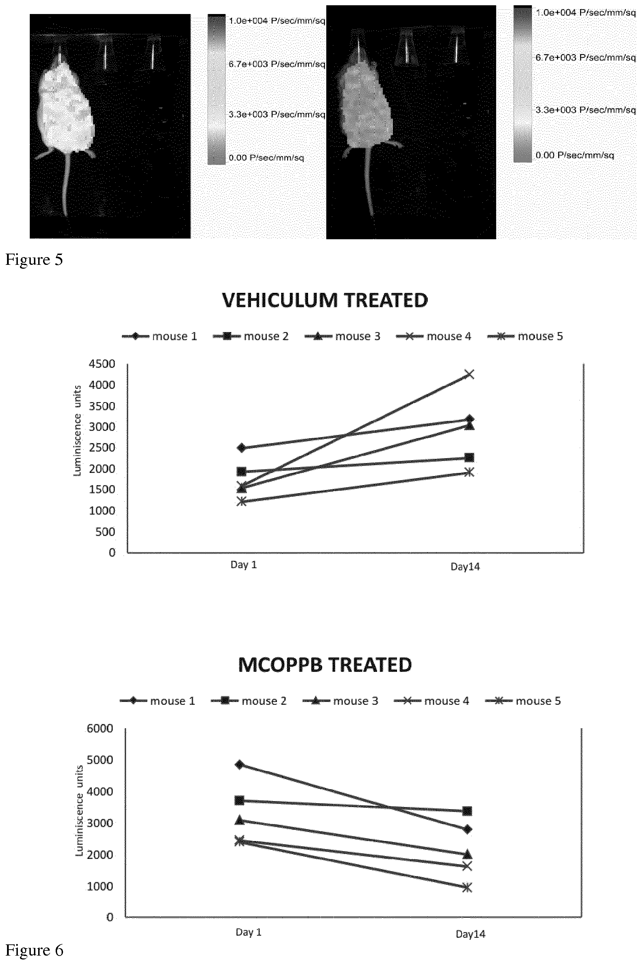

Reporter GMO mouse with integrated luciferase reporter under p16 promoter allows monitoring of aging on the molecular level by an increase of luminescence signal due to the accumulation of senescent cells (this mouse model is described in details in: Cell. 2013 Jan 17;152(1-2):340-51). Application of senolytics targets the p16 positive cells and leads to decrease in the luminescent signal. Naturally aged reporter mice were treated by the MCOPPB compound and luminescence signal level was measured over time. Mice treated by MCOPPB indeed show decrease in the luminescence signal as compared to controls as depicted in figure 5 and 6, thus confirming senolytic effect of MCOPPB *in-vivo.*

## Claims

1. 1-[1-(1-methylcyclooctyl)-4-piperidinyl]-2-[(3R)-3-piperidinyl]-1H-benzimidazole of formula 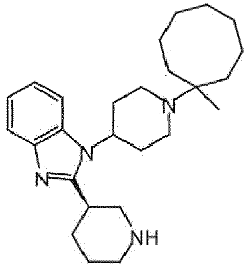 for use in a method of treatment of a condition involving accumulation of senescent cells.

2. The compound for use according to claim 1, wherein the condition involving accumulation of senescent cells is selected from: advanced age-related disorders caused by natural accumulation of senescent cells, inherited premature aging syndromes, disorders caused by aberrant accumulation of senescent cells during ontogenesis of the organism, accumulation of senescent cells induced by environmental poisoning, accumulation of senescent cells induced by chemotherapy, local accumulation of senescent cells in tissues and organs induced by therapeutic or accidental irradiation by ionizing radiation, accumulation of senescent cells in the skin induced by UV light, accumulation of senescent cells induced by oncogenic transformation of cells and/or other behavioral factors.

3. The compound for use according to claim 1, wherein the condition involving accumulation of senescent cells is selected from: cancer, cardiovascular, neurology and immune related diseases.

4. The compound for use according to claim 1, wherein the senescent cells are selected from: muscle cells, skin cells, haematopoietic cells, neural cells, stem cells, cancer cells.

5. The compound for use according to any one of the preceding claims, wherein the patient to be treated is a mammal, in particular selected from humans, dogs, cats, horses, cows, goats, sheep and pigs.

6. The compound for use according to any one of the preceding claims, wherein the compound is administered at a dose of about 1-100 mg/kg/day, preferably 5-50 mg/kg/day, more preferably 10-20 mg/kg/day.
